# EUROPEAN PATENT SPECIFICATION

(11) **EP 2 605 743 B1**
(45) Date of publication and mention of the grant of the patent: **20.05.2015**
(21) Application number: 11724257.8
(22) Date of filing: 10.06.2011
(51) Int. Cl.: A61K 8/31, A61Q 5/12, A61K 8/92

(54) **HAIR CONDITIONING COMPOSITION**
HAARPFLEGEZUSAMMENSETZUNG
COMPOSITION DE SOIN CAPILLAIRE

(30) Priority: 20.08.2010 EP 10173505
(43) Date of publication of application: 26.06.2013
(73) Proprietor: Unilever PLC, London, Greater London EC4Y 0DY (GB); Unilever N.V., 3013 AL Rotterdam (NL)
(72) Inventor: IVANOVA, Katya, Wirral Merseyside CH63 3JW (GB)
(74) Representative: Chisem, Janet
(86) International application number: PCT/EP2011/059727
(87) International publication number: WO 2012/022516

(56) References cited:
- EP-A1- 0 530 974
- EP-A1- 1 520 575
- WO-A1-01/00140
- WO-A1-01/95866
- WO-A1-2006/066656
- WO-A1-2006/066668
- WO-A1-2006/066703
- WO-A1-2006/066710
- WO-A2-2008/003677
- CH-A- 272 709
- US-A1- 2003 086 897

## Description

The present invention relates to a hair conditioning composition comprising an oil blend.

WO 01/95866 (Unilever) discloses a hair oil comprising mineral oil and coconut oil. This disclosure demonstrates that adding mineral oil to coconut oil reduces greasiness and increases penetration of total oil in hair.

J. Cosmetic Sci., 56, 283-295 (September/October 2005) discloses that oils applied on hair leave an oily film and oil makes capillary bridges between hair fibres thus 'adhering' fibres together. With time the thickness of the oil film decreases and the adhesion reduces due to the oil being absorbed by the hair (only penetrating oils show this effects). The adhesion between the fibres and the thick oil film lead to greasy feel and weigh down look.

Despite the prior art there remains a need for improved conditioning compositions.

We have surprisingly found that adding coconut oil to mineral oil in a conditioner formulation weighs hair down less compared to mineral oil alone. It is even more surprising that using combination of oil and silicone hair leads to less weigh down hair and more smooth hair ends compared to using silicone alone.

Accordingly, the present invention provides a composition according to claim 1.

### (i) First oily component

The first oily component may be present in the hair oil blends of the invention as a single material or as a blend.

The total content of first oily component in the hair oil blends of the invention suitably ranges from 10% to 95%, preferably from 20% to 80%, by weight based on total weight of the composition.

### (ii) Hydrocarbon Oil

Suitable hydrocarbon oils include cyclic hydrocarbons, straight chain aliphatic hydrocarbons (saturated or unsaturated), and branched chain aliphatic hydrocarbons (saturated or unsaturated). Straight chain hydrocarbon oils will typically contain from about 6 to about 16 carbon atoms, preferably from about 8 up to about 14 carbon atoms. Branched chain hydrocarbon oils can and typically may contain higher numbers of carbon atoms, e.g. from about 6 up to about 20 carbon atoms, preferably from about 8 up to about 18 carbon atoms.

Suitable hydrocarbon oils of the invention will generally have a viscosity at ambient temperature (25 to 30°C) of from 0.0001 to 0.5 Pa.s, preferably from 0.001 to 0.05 Pa.s, more preferably from 0.001 to 0.02 Pa.s as measured by a Carri-Med CSL2 100 controlled stress rheometer, from TA Instruments Inc., New Castle, Delaware (USA).

A preferred hydrocarbon oil is light mineral oil. Mineral oils are clear oily liquids obtained from petroleum oil, from which waxes have been removed, and the more volatile fractions removed by distillation. The fraction distilling between 250°C to 300°C is termed mineral oil, and it consists of a mixture of hydrocarbons, in which the number of carbon atoms per hydrocarbon molecule generally ranges from C₁₀ to C₄₀. Mineral oil may be characterised in terms of its viscosity, where light mineral oil is relatively less viscous than heavy mineral oil, and these terms are defined more specifically in the U.S. Pharmacopoeia, 22nd revision, p. 899 (1990). A commercially available example of a suitable light mineral oil for use in the invention is Sirius M40 (carbon chain length) C₁₀-C₂₈, mainly C₁₂-C₂₀, viscosity 4.3 x 10⁻³ Pa.s), available from Silkolene.

Other hydrocarbon oils that may be used in the invention include relatively lower molecular weight hydrocarbons including linear saturated hydrocarbons such a tetradecane, hexadecane, and octadecane, cyclic hydrocarbons such as dioctylcyclohexane (e.g. CETIOL S from Henkel), branched chain hydrocarbons (e.g. ISOPAR L and ISOPAR V from Exxon Corp.).

The hydrocarbon oil may be present in hair oil blends of the invention as a single material or as a blend.

The total content of hydrocarbon oil in hair oil blends of the invention suitably ranges from 5% to 90%, preferably from 20% to 80%, by weight based on total weight of the hair oil blend.

The first oily component: hydrocarbon oil weight ratio in compositions of the invention may suitably range from 90:10 to 10:90, most preferably from 80:20 to 20:80. Particularly preferred are blends of [coconut oil and/or sunflower oil and/or almond oil] and light mineral oil, in which the [coconut oil and/or sunflower oil and/or almond oil]: light mineral oil weight ratio is 60:40.

### Other Oily Materials

Other oily materials may also be present in combination with the hydrocarbon oils and glyceride fatty esters in hair oil blends of the invention. Suitable additional oily materials include other fatty esters, fatty alcohols and fatter ethers.

In general, fatty esters, fatty alcohols and ethers are characterised by having at least 10 carbon atoms, and include esters and ethers with hydrocarbyl chains derived from fatty acids or alcohols, e.g., monocarboxylic acid esters, polyhydric alcohol esters, and di- and tricarboxylic acid esters and ethers, and fatty alcohols with a carbon chain carbon chain length of between 10 and 18, e.g. lauryl alcohol, cetyl alcohol or cetostearyl alcohol.

Examples include isopropyl myristate, isopropyl palmitate, cetearyl isononanoate, cetearyl octanoate, diethylene glycol monoethyl ether oleate, dicaprylyl ether, caprylic acid/capric acid propylene glycol diester and mixtures of any of the above.

The total content of other oily material in compositions of the invention suitably ranges from 0.01 % to 50%, preferably from 1.0% to 20%, by weight based on total weight of the hair oil blend.

The oil blend is present in the composition at from 0.01 to 10% wt. of the composition, more preferably at from 0.1 to 2% wt.

### (iii) Cationic Surfactants

Suitable conditioning surfactants are selected from cationic surfactants, used singly or in admixture. Examples include quaternary ammonium hydroxides or salts thereof, e.g. chlorides.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as DEHYQUART, ex Henkel.

In conditioners of the invention, the level of cationic surfactant is preferably from 0.01 to 10%, more preferably 0.05 to 5%, most preferably 0.1 to 2% by weight of the composition.

### Product Form

Compositions of this invention are preferably dedicated hair conditioning compositions and as so comprise less than 5% wt. anionic surfactant, preferably less than 5% wt. cleansing surfactant, more preferably less than 2% wt. anionic surfactant, preferably less than 2% wt. cleansing surfactant, and most preferably no cleansing surfactant.

### Optional Ingredients

Compositions of this invention may contain any other ingredient normally used in hair conditioning formulations. These other ingredients may include, viscosity modifiers, preservatives, colouring agents, polyols such as glycerine and polypropylene glycol, chelating agents such as EDTA, antioxidants such as BHT (butylhydroxytoluene), vitamin E acetate, fragrances, antimicrobials and sunscreens and lipid soluble ingredients e.g. fatty acids or sterols. Each of these ingredients will be present in an amount effective to accomplish its purpose.

Generally these optional ingredients are included individually at a level of up to about 5% by weight of the total composition.

Preferably, the composition comprises a conditioning active selected from fatty alcohols and conditioning silicones.

Conditioners of the invention advantageously incorporate a fatty alcohol material. The combined use of fatty alcohol materials and cationic surfactants in conditioning compositions is believed to be especially advantageous, because this leads to the formation of a lamellar phase, in which the cationic surfactant is dispersed. Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol material in conditioners of the invention is conveniently from 0.01 to 10%, preferably from 0.1 to 5% by weight of the composition. The weight ratio of cationic surfactant to fatty alcohol is suitably from 10:1 to 1:10, preferably from 4:1 to 1:8, optimally from 1:1 to 1:4.

Silicone is a particularly preferred ingredient in hair treatment compositions of the invention. In particular, conditioners of the invention will preferably also comprise emulsified particles of silicone, for enhancing conditioning performance. The silicone is insoluble in the aqueous matrix of the composition and so is present in an emulsified form, with the silicone present as dispersed particles.

Suitable silicones include polydiorganosiloxanes, in particular polydimethylsiloxanes which have the CTFA designation dimethicone. Also suitable for use compositions of the invention are polydimethyl siloxanes having hydroxyl end groups, which have the CTFA designation dimethiconol. Also suitable for use in compositions of the invention are silicone gums having a slight degree of cross-linking, as are described for example in WO 96/31188. These materials can impart body, volume and stylability to hair, as well as good wet and dry conditioning.

The viscosity of the emulsified silicone itself (not the emulsion or the final hair conditioning composition) is typically at least 10,000 cst. In general we have found that conditioning performance increases with increased viscosity. Accordingly, the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in conditioners of the invention will typically have an average silicone particle size in the composition of less than 30, preferably less than 20, more preferably less than 10 microns. We have found that reducing the particle size generally improves conditioning performance. Most preferably the average silicone particle size of the emulsified silicone in the composition is less than 2 microns, ideally it ranges from 0.01 to 1 micron. Silicone emulsions having an average silicone particle size of ≤ 0.15 microns are generally termed microemulsions.

Particle size may be measured by means of a laser light scattering technique, using a 2600D Particle Sizer from Malvern Instruments.

Suitable silicone emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

A further preferred class of silicones for inclusion in conditioners of the invention are amino functional silicones. By "amino functional silicone" is meant a silicone containing at least one primary, secondary or tertiary amine group, or a quaternary ammonium group.

Examples of suitable amino functional silicones include:
(i) polysiloxanes having the CTFA designation "amodimethicone", and the general formula:

   HO-[Si(CH₃)₂-O-]ₓ-[Si(OH)(CH₂CH₂CH₂-NH-CH₂CH₂NH₂)-O-]_{y}-H

   in which x and y are numbers depending on the molecular weight of the polymer, generally such that the molecular weight is between about 5,000 and 500,000.
(ii) polysiloxanes having the general formula:

   R^{'}ₐG₃₋ₐ-Si(OSiG₂)ₙ-(OSiG_{b}R^{'}_{2-b})ₘ-O-SiG₃₋ₐ-R^{'}ₐ

   in which:
   G is selected from H, phenyl, OH or C₁₋₈ alkyl, e.g. methyl;
   a is 0 or an integer from 1 to 3, preferably 0;
   b is 0 or 1, preferably 1;
   m and n are numbers such that (m + n) can range from 1 to 2000, preferably from 50 to 150;
   m is a number from 1 to 2000, preferably from 1 to 10;
   n is a number from 0 to 1999, preferably from 49 to 149, and
   R is a monovalent radical of formula -C_{q}H_{2q}L in which q is a number from 2 to 8 and L is an aminofunctional group selected from the following:
      -NR^{"}-CH₂-CH₂-N(R^{"})₂
      -N(R^{"})₂
      -N⁺(R^{"})₃A⁻
      -N⁺H(R^{"})₂A⁻
      -N⁺H₂(R^{"})A⁻
      -N(R^{"})-CH₂-CH₂-N⁺H₂(R^{"})A⁻
   in which R is selected from H, phenyl, benzyl, or a saturated monovalent hydrocarbon radical, e.g. C₁₋₂₀ alkyl, and;
   A is a halide ion, e.g. chloride or bromide.

   Suitable amino functional silicones corresponding to the above formula include those polysiloxanes termed "trimethylsilylamodimethicone" as depicted below, and which are sufficiently water insoluble so as to be useful in compositions of the invention:

   Si(CH₃)₃-O-[Si(CH₃)₂-O-]ₓ-[Si(CH₃)(R -NH-CH₂CH₂NH₂)-O-]_{y} -Si(CH₃)₃

   wherein x + y is a number from about 50 to about 500, and wherein R is an alkylene group having from 2 to 5 carbon atoms. Preferably, the number x + y is in the range of from about 100 to about 300.
(iii) quaternary silicone polymers having the general formula:

   {(R¹)(R²)(R³)N⁺CH₂CH(OH)CH₂O(CH₂)₃[Si(R⁴)(R⁵)-O-]ₙ-Si(R⁶)(R⁷)-(CH₂)₃-O-CH₂CH(OH)CH₂N⁺(R⁸)(R⁹)(R¹⁰)}(X⁻)₂

   wherein R¹ and R¹⁰ may be the same or different and may be independently selected from H, saturated or unsaturated long or short chain alk(en)yl, branched chain alk(en)yl and C₅-C₈ cyclic ring systems;
   R² thru' R⁹ may be the same or different and may be independently selected from H, straight or branched chain lower alk(en)yl, and C₅-C₈ cyclic ring systems;
   n is a number within the range of about 60 to about 120, preferably about 80, and
   X- is preferably acetate, but may instead be for example halide, organic carboxylate, organic sulphonate or the like.

   Suitable quaternary silicone polymers of this class are described in EP-A-0 530 974.

Amino functional silicones suitable for use in conditioners of the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance.

The viscosity of the amino functional silicone is not particularly critical and can suitably range from about 100 to about 500,000 cst.

Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones).

Also suitable are emulsions of amino functional silicone oils with non ionic and/or cationic surfactant.

Suitably such pre-formed emulsions will have an average amino functional silicone particle size in the composition of less than 30, preferably less than 20, more preferably less than 10 microns. Again, we have found that reducing the particle size generally improves conditioning performance. Most preferably the average amino functional silicone particle size in the composition is less than 2 microns, ideally it ranges from 0.01 to 1 micron. Silicone emulsions having an average silicone particle size of ≤ 0.15 microns are generally termed microemulsions.

Pre-formed emulsions of amino functional silicone are also available from suppliers of silicone oils such as Dow Corning and General Electric. Specific examples include DC929 Cationic Emulsion, DC939 Cationic Emulsion, and the non-ionic emulsions DC2-7224, DC2-8467, DC2-8177 and DC2-8154 (all ex Dow Corning).

An example of a quaternary silicone polymer useful in the present invention is the material K3474, ex Goldschmidt.

The total amount of silicone incorporated into compositions of the invention depends on the level of conditioning desired and the material used. A preferred amount is from 0.01 to about 10% by weight of the total composition although these limits are not absolute. The lower limit is determined by the minimum level to achieve conditioning and the upper limit by the maximum level to avoid making the hair and/or skin unacceptably greasy.

We have found that a total amount of silicone of from 0.3 to 5%, preferably 0.5 to 3%, by weight of the total composition is a suitable level.

Other ingredients may include viscosity modifiers, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally these optional ingredients are included individually at a level of up to about 5% by weight of the total composition.

Preferably, compositions of this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts.
(ii) hair fibre benefit agents. Examples are: ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
   - free fatty acids, for cuticle repair and damage prevention. Examples are branched chain fatty acids such as 18-methyleicosanoic acid and other homologues of this series, straight chain fatty acids such as stearic, myristic and palmitic acids, and unsaturated fatty acids such as oleic acid, linoleic acid, linolenic acid and arachidonic acid. A preferred fatty acid is oleic acid. The fatty acids may be added singly, as mixtures, or in the form of blends derived from extracts of, e.g. lanolin.

Mixtures of any of the above active ingredients may also be used.

Preferably, the composition is a rinse-off conditioning composition.

The invention is further illustrated by way of the following Examples, in which all percentages are by weight based on total weight unless otherwise stated.

### EXAMPLE

The following formulations were made by standard processes. Formulation 1 is an inventive formulation while formulations A and B are comparative formulations.

| | **1** | **A** | **B** |
|---|---|---|---|
| **Ingredient** | **% wt.** | | |
| Stearamidopropyl dimethyl amine | 1.25 | 1.25 | 1.25 |
| Behenyl trimonium chloride | 1.25 | 1.25 | 1.25 |
| Cetyl Stearyl alcohol | 5.00 | 5.00 | 5.00 |
| Buffer | 0.4 | 0.4 | 0.4 |
| Silicone | 2.5 | 2.5 | 2.5 |
| | | | |

| **Oil Blend** | | | |
|---|---|---|---|
| Coconut oil | 0.9 | | |
| Light mineral oil | 0.525 | | 1.5 |
| Almond oil | 0.075 | | 0.075 |
| | | | |
| Preservatives | Trace | Trace | Trace |
| Colourants | Trace | Trace | Trace |
| Water | To 100 | To 100 | To 100 |

### EXAMPLE 2

In a blind monadic test users were asked to compare the formulations according to Example 1 for the weight of their hair after using each product for three times a week for two weeks.

The results show that the composition according to Example 1 performed better than that according to the comparatives.

| | 1 | A | B |
|---|---|---|---|
| Hair heaviness | 4.3 | 4.2 | 4.1 |

Formulation 1 also wins against A and B on the two top boxes which means that the highest percentage of consumers ticking the top two boxes (i.e. 4 and 5) was highest for Example 1 over A and B.

## Claims

1. A rinse-off hair conditioning composition comprising a cationic surfactant and an oil blend, said blend comprising:
(i) from 10% to 95%, by weight based on total weight, of a first oily component which is selected from coconut oil, sunflower oil, almond oil and mixtures thereof, and
(ii) from 5% to 90%, by weight based on total weight, of a second oily component which is one or more hydrocarbon oils of average carbon chain length less than 20 carbon atoms,
wherein, the oil blend is present in the composition at from 0.1 to 2% wt.

2. Composition according to claim 1, in which the total content of the first oily component ranges from 20% to 80% by weight based on total weight of the oil blend.

3. Composition according to any one of claims 1 or 2, in which the hydrocarbon oil is light mineral oil.

4. Composition according to any one of claims 1 to 3, in which the total content of hydrocarbon oil ranges from 20% to 80% by weight based on total weight of the oil blend.

5. Composition according to any one of claims 1 to 4, in which the first oily component: hydrocarbon oil weight ratio ranges from 90:10 to 10:90 and most preferably from 80:20 to 20:80.

## Patentansprüche

1. Haarkonditionierzusammensetzung zum Ausspülen,
die ein kationisches Tensid und ein Ölgemisch aufweist, wobei das Gemisch Folgendes aufweist:
i) 10 bis 95 Gew.-%, auf das Gesamtgewicht bezogen, einer ersten Ölkomponente, die aus Kokosnussöl, Sonnenblumenöl, Mandelöl und Gemischen davon ausgewählt ist, und
ii) 5 bis 90 Gew.-%, auf das Gesamtgewicht bezogen, einer zweiten Ölkomponente, die ein oder mehrere Kohlenwasserstofföle mit einer durchschnittlichen Länge der Kohlenstoffkette von weniger als 20 Kohlenstoffatomen ist,
wobei das Ölgemisch mit 0,1 bis 2 Gew.-% in der Zusammensetzung vorliegt.

2. Zusammensetzung nach Anspruch 1,
wobei der Gesamtgehalt der ersten Ölkomponente im Bereich von 20 bis 80 Gew.-% liegt, und zwar auf das Gesamtgewicht des Ölgemischs bezogen.

3. Zusammensetzung nach einem der Ansprüche 1 oder 2,
wobei das Kohlenwasserstofföl leichtes Mineralöl ist.

4. Zusammensetzung nach einem der Ansprüche 1 bis 3,
wobei der Gesamtgehalt des Kohlenwasserstofföls im Bereich von 20 bis 80 Gew.-% liegt, und zwar auf das Gesamtgewicht des Ölgemischs bezogen.

5. Zusammensetzung nach einem der Ansprüche 1 bis 4,
wobei das Gewichtsverhältnis von erster Ölkomponente : Kohlenwasserstofföl im Bereich von 90:10 bis 10:90 und besonders bevorzugt von 80:20 bis 20:80 liegt.

## Revendications

1. Composition d'après-shampoing à rincer comprenant un tensioactif cationique et une combinaison d'huile, ladite combinaison comprenant :
(i) de 10 % à 95 %, en poids rapporté au poids total, d'un premier constituant huileux qui est choisi parmi l'huile de noix de coco, l'huile de tournesol, l'huile d'amande et des mélanges de celles-ci, et
(ii) de 5 % à 90 %, en poids rapporté au poids total, d'un second constituant huileux qui est une ou plusieurs huiles hydrocarbonées de longueur de chaîne carbonée moyenne inférieure à 20 atomes de carbone,
dans laquelle, la combinaison d'huile est présente dans la composition à de 0,1 à 2 % en poids.

2. Composition selon la revendication 1, dans laquelle la teneur totale du premier constituant huileux est de 20 % à 80 % en poids rapporté au poids total de la combinaison d'huile.

3. Composition selon l'une quelconque des revendications 1 ou 2, dans laquelle l'huile hydrocarbonée est une huile minérale légère.

4. Composition selon l'une quelconque des revendications 1 à 3, dans laquelle la teneur totale en huile hydrocarbonée est de 20 % à 80 % en poids rapportée au poids total de la combinaison d'huile.

5. Composition selon l'une quelconque des revendications 1 à 4, dans laquelle le rapport massique premier constituant huileux : huile hydrocarbonée est de 90:10 à 10:90 et encore mieux de 80:20 à 20:80.
